**Europäisches Patentamt**

**European Patent Office**

**Office européen des brevets**

(19)

(11) Veröffentlichungsnummer: **0 342 369**

**A2**

(12) **EUROPÄISCHE PATENTANMELDUNG**

(21) Anmeldenummer: **89106960.1**

(22) Anmeldetag: **19.04.89**

(51) Int. Cl.4: **A61K 7/16**

(30) Priorität: **14.05.88 DE 3816522**

(43) Veröffentlichungstag der Anmeldung:
**23.11.89 Patentblatt 89/47**

(84) Benannte Vertragsstaaten:
**AT BE CH DE ES FR GB GR IT LI LU NL SE**

(71) Anmelder: **Biodyn AG**
**Industriestr. 31**
**CH-8305 Dietlikon(CH)**

(72) Erfinder: **Lembke, Andreas, Prof.Dr.Dr.**
**Eutiner Strasse 1**
**D-2420 Eutin-Sielbeck(DE)**
Erfinder: **Mader, Helmut J.**
**Steiner Strasse 13**
**D-8543 Hilpoltstein(DE)**
Erfinder: **Strobel, Hans Joachim, Dr.**
**Opfikoner Strasse 3**
**CH-8303 Bassersdorf(CH)**

(74) Vertreter: **Hach, Hans Karl, Dr.**
**Tarunstrasse 23**
**D-6950 Mosbach-Waldstadt(DE)**

(54) **Schutzmittel gegen Karies.**

(57) Ein Schutzmittel gegen Karies besteht aus einem verträglichen Träger und einem Zusatz von Galactose.

EP 0 342 369 A2

## SCHUTZMITTEL GEGEN KARIES

Die Erfindung betrifft ein Schutzmittel gegen Karies.

Karies wird hervorgerufen durch säurebildende Bakterien, die mit Haftrezeptoren Halt an der Zahnoberfläche finden, und deren Lebensbedingungen begünstigt werden, vor allem durch Saccharose, weil diese für die Bakterien ein günstiges Nahrungsmittel ist und zudem dazu neigt, an den Zähnen zu haften.

Aus der EU 01844121/A2 ist es bekannt, saccharosehaltigen Nahrungsmitteln und saccharosehaltigen Pharmazeutika Galactose zuzusetzen, weil Galactose die Rezeptoren belegt, mit denen die Karies hervorrufenden Bakterien an der Zahnoberfläche haften.

Aufgabe der Erfindung ist es, mit Galactose der Kariesbildung entgegenzuwirken, und zwar unabhängig von der Aufnahme saccharosehaltiger Nahrungsmittel beziehungsweise saccharosehaltiger Pharmazeutika wie nach dem genannten Stand der Technik vorgesehen.

Die Erfindung löst diese Aufgabe durch, ein Schutzmittel mit einem zur oralen, humanen Applikation verträglichen Träger, der den Benutzer zu einer längeren Verweilzeit im Mund zwingt und dem Galactose in feinster Verteilung beigemischt ist.

Der Benutzer kann vor, nach und während der Nahrungsaufnahme das Schutzmittel einnehmen und führt dadurch seinen Zähnen beziehungsweise der Umgebung seiner Zähne Galactose zu, die, bedingt durch die lange Verweildauer des Trägers, über einen längeren Zeitraum verteilt im Mund in Lösung geht und entsprechend intensiv im Bereich der Zähne gegen Kariesbildung wirksam ist.

Der Träger sollte allenfalls sehr wenig, besser gar keinen, Zucker, ausgenommen Galactose und insbesondere allenfalls sehr wenig, besser gar keine, Saccharose enthalten, weil alle diese Zucker, vor allem Saccharose, die Kariesbildung begünstigen, eine Wirkung, die gerade durch die Erfindung vermieden werden soll.

Dem trägt eine Weiterbildung der Erfindung Rechnung, die dadurch gekennzeichnet ist, daß der Gehalt des Trägers an Saccharose 0 bis 2, vorzugsweise 0 TGT auf 100 TGT Trägersubstanz beträgt und/oder daß der Gehalt des Trägers an Zucker, ausgenommen Galactose, 0 bis 1, vorzugsweise 0 TGT auf 100 TGT Trägersubstanz beträgt.

Besonders wirkungsvoll ist ein solches Schutzmittel, wenn es vor dem Einschlafen eingenommen wird, weil dadurch die sich über die lange Nachtzeit erstreckende kariesfördernde Wirkung der fraglichen Bakterien behindert wird.

Es sind verschiedene Ausgestaltungen des Trägers vorteilhaft. Bevorzugt ist eine Ausgestaltung, bei der der Träger eine Zahnpasta, ein Mundwasser, ein Kaugummi oder dergleichen Nicht-Nahrungsmittel ist. Ein solcher Träger bietet den Vorteil, daß die Schutzwirkung auch erzielt werden kann, ohne daß Nahrungsmittel oder Pharmazeutika heruntergeschluckt werden müssen.

Eine erste Ausgestaltung ist dadurch gekennzeichnet, daß der Träger eine Zahnpasta ist und daß 1 bis 10 TGT, vorzugsweise 3 TGT, Galactose auf 100 TGT Trägersubstanz eingesetzt sind, wobei vorzugsweise der Träger aus

40 bis 50 TGT Putzkörper, vorzugsweise feinstkörnigem Kalziumcarbonat,

10 bis 30 TGT Feuchthaltemittel, vorzugsweise Glycerin,

1 bis 2 TGT Binde- und Verdickungsmittel, vorzugsweise Zelluloseester,

0,5 bis 2 TGT Tenside, vorzugsweise Natriumlaurylsulfat,

1 bis 2 TGT sonstige Wirkstoffe, vorzugsweise Vitamine und Mineralsalze,

1 bis 2 TGT Aromastoffe, vorzugsweise ätherische Öle,

0,1 bis 1 TGT Süßstoffe, vorzugsweise Saccharin-Natrium,

0,1 bis 1 TGT Konservierungsstoffe, vorzugsweise Benzoesäure, und gegebenenfalls

1 bis 15 TGT weiteren Zusätzen, wie Binde- und Verdickungsmitteln, Tenside, Wirkstoffe, Aromastoffe, Süßstoffe und Konservierungsstoffe, besteht.

Mit einer Zahnpasta wird die Galactose intensiv im Zahnbereich verteilt und kann dort wirksam werden, wobei der Umstand, daß im allgemeinen nach der Mahlzeit die Zähne geputzt werden, von besonderem Vorteil ist, weil er den Nachwirkungen der möglicherweise zuckerhaltigen Nahrungsaufnahme entgegenwirkt.

Eine zweite bevorzugte Ausgestaltung ist dadurch gekennzeichnet, daß der Träger aus Kaugummi besteht und daß 50 bis 400, vorzugsweise 300, TGT Galactose auf 100 TGT Trägersubstanz eingesetzt sind, wobei der Träger vorzugsweise aus Chiclegummi und geschmacksbildenden Zusatzstoffen, wie Eukalyptus-Öl, Süßstoff, Fenchel-Öl, Balsam und dergleichen besteht.

Ein Kaugummi verweilt sehr lange im Mund, so daß die Zeit der Abgabe der Galactose verhältnismäßig lang ist. Das macht es möglich, einen verhältnismäßig hohen Prozentsatz an Galactose beizumischen. Im übrigen bietet auch das Kaugummi die schnelle Verfügbarkeit der Galactose wie die Kautablette.

Eine weitere bevorzugte Ausgestaltung ist dadurch gekennzeichnet, daß der Träger Wasser, vorzugsweise Mundwasser, ist und daß 1 bis 10, vorzugsweise 5 TGT Galactose auf 100 ccm (Kubikzentimeter) Wasser gelöst sind, wobei dem Mundwasser außerdem noch Desinfektionsmittel, vorzugsweise 0,5 bis 2,0 g ätherisches Öl, beispielsweise Pfefferminzöl auf 100 ccm Wasser beigemischt sein kann.

Die Erfindung ist auch anwendbar in Verbindung mit Trägersubstanzen, die zu den Nahrungsmitteln gehören und hinuntergeschluckt werden, wiewohl das nicht so vorteilhaft ist wie der Einsatz von Trägern, die aus Nicht-Nahrungsmittel bestehen und deshalb nicht hinuntergeschluckt werden, denn man wünscht ja in vielen Fällen, die angestrebte Schutzwirkung zu erzielen, ohne daß dabei der Magen durch Nahrungsaufnahme oder dergleichen belastet wird, was insbesondere vor dem Einschlafen für viele Anwendungsfälle nicht wünschenswert ist.

Eine dementsprechende bevorzugte Ausgestaltung ist dadurch gekennzeichnet, daß der Träger eine Kautablettenmasse ist und daß 3 bis 200 TGT, vorzugsweise 100 TGT, Galactose auf 100 TGT Trägersubstanz eingesetzt sind, wobei der Träger vorzugsweise im wesentlichen aus Zucker und/oder Zuckeraustauschstoff und einem Direktkomprimiermittel, vorzugsweise Calciumhydrogenphosphat (zum Beispiel das unter dem Warenzeichen "Emkompress" bekannte), besteht und mit der Galactose vermischt tablettiert ist.

Als Direktkomprimiermittel kommen auch in Betracht: Lactose (wie sie beispielsweise unter der Warenbezeichnung "Tablettose" bekannt ist) und Polyglucose (wie sie bei spielsweise unter der Warenbezeichnung "Emdex" bekannt ist). Calciumhydrogenphosphat ist als Direktkomprimiermittel hier deshalb bevorzugt, weil es per se zur Prophylaxe der Karies dient und darüberhinaus aufgrund seines günstigen pH-Wertes synergistisch die Kariesprophylaxe der Galactose unterstützt.

Eine Kautablette hat den Vorteil, daß sie leicht greifbar ist und daß beim Verkauen die Galactose im Zahnbereich austritt. Es kann davon ausgegangen werden, daß hinreichende Reste der Galactose auch nach dem Hinunterschlucken der Kausubstanz im Mund verbleiben.

Eine weitere bevorzugte Ausgstaltung ist dadurch gekennzeichnet, daß der Träger ein Lutschbonbon vorwiegend aus Zuckeraustauschstoff, wie Sorbit, Xylit, Manit oder dergleichen, ist und daß 1 bis 10, vorzugsweise 3 TGT Galactose auf 100 TGT Trägersubstanz eingesetzt sind

Auch bei einem Lutschbonbon erstreckt sich das In-Lösung-Gehen der Galactose über einen verhältnismäßig langen Zeitraum, so daß man auch beim Lutschbonbon die Zusatzmenge an Galactose relativ hoch bemessen kann.

Die Erfindung wird nun anhand einiger Beispiele näher erläutert.

BEISPIEL 1: Zahnpasta

Die Zahnpasta besteht aus
45 TGT feinstkörnigem Kalziumcarbonat,
20 TGT Glycerin,
1,5 TGT Zelluloseester,
1,7 TGT Natriumlaurylsulfat,
1,5 TGT Vitamine und Mineralsalze,
1,5 TGT ätherische Öle,
0,6 TGT Saccharin-Natrium,
0,6 TGT Benzoesäure,
50 Gewichtsteile Wasser und
7 TGT Galactose.

Die festen Bestandteile sind feinvermahlen auf eine Korngröße von $1 . 10^{-3}$ mm (Millimeter) bis $10 . 10^{-3}$ mm und mit den übrigen Bestandteilen feinvermischt zu einer pastosen Masse.

BEISPIEL 2: Kautablette

100 TGT Sorbit, gemahlen auf eine Korngröße von $1 . 10^{-2}$ mm bis $1 . 10^{-1}$ mm sind vermischt mit 10 TGT Galactose, feinvermahlen auf $1 . 10^{-3}$ mm. Die Substanzen sind intensiv miteinander vermischt und in einer Tablettiermaschine zu festen Tabletten von je 3 g (Gramm) Gewicht verfestigt.

BEISPIEL 3: Kaugummi

Eine plastische Masse besteht aus 175 TGT Chiclegummi, 6 TGT Glyzerin, 6 TGT Balsam, 3 TGT Alaun und 570 TGT Galactose. Das Alaun und die Galactose sind feinkörnig vermahlen auf eine Korngröße von $5 . 10^{-2}$ mm bis $7 . 10^{-2}$ mm. Die Substanzen sind intensiv miteinander vermischt. Ein Kaugummi besteht aus einer kugelförmigen 3 g schweren Portion dieser plastischen Masse, die mit einem 0,5 mm starken Überzug aus Sorbit umgossen ist.

BEISPIEL 4: Lutschbonbon

100 TGT Sorbit sind mit 7 TGT Galactose in Wasser gelöst und auskristallisiert. Die kristalline Masse ist zerbrochen in Teilchen von 1 bis 2 g Gewicht.

BEISPIEL 5: Mundwasser

Auf 100 ccm Wasser sind 5 g Galactose gelöst und 2 g Pfefferminzöl emulgiert.

## BEISPIEL 6: Kautablette

Saccharosesirup mit einem Wassergehalt von 20% (Prozent) und wässrige Galactoselösung werden vermischt. Der Einsatz erfolgt mit 1 kg (Kilogramm) Trockengewicht Saccharose und 50 g Trockengewicht Galactose. Das sind 5 TGT Galactose auf 100 TGT Trägersubstanz, die hier aus Saccharose besteht. Die Mischung wird in einem Rotationsverdampfer eingeengt. Die nicht kristallisierte, noch leicht wasserhaltige Mischung wird durch Auftragen auf eine Kühlwalze zum Erstarren gebracht. Die erstarrte Schmelze wird in einem Brecher zerkleinert und anschließend in Partikel mit einem Durchmesser von 1 bis 2 mm (Millimeter) gemahlen. Die so erhaltenen Pellets werden im Trockengewichtsverhältnis 1:1 mit Calciumhydrogenphosphat gemischt und dann wird die Mischung direkt zu Tabletten gepreßt.

Das Beispiel 6 ist abänderbar, indem statt der Saccharose in der gleichen Menge ein Zuckeraustauschstoff, wie beispielsweise Sorbit, Manit oder Xylit eingesetzt wird.

## BEISPIEL 7: Kautablette

1 kg Calciumhydrogenphosphat mit Korngröße kleiner als 1 mm wird in einem Dragierkessel vorgelegt und unter ständigem Rotieren wird intermittierend eine wässrige Galactoselösung mit insgesamt 1 kg Trockengewicht Galactose aufgesprüht. Die Masse wird dann durch Einleiten von Heißluft getrocknet. Das so gewonnene Pulver wird direkt zu Tabletten gepreßt.

## Weitere Beispiele als Abänderungen der vorgenannten Beispiele

Die Beispiele 1 bis 7 sind abänderbar, indem zusätzlich zur Galactose künstlicher Süßstoff, wie Cyclamat, Saccharinat und/oder Aspartame und/oder Zuckeraustauschstoff, wie Sorbit, Manit, Xylit oder dergleichen, zugesetzt wird, und zwar vorzugsweise mit der gleichen Technik wie die Galactose zugesetzt wird. Die Menge an zugesetztem künstlichen Süßstoff und/oder Zuckeraustauschstoff wird dabei so gewählt, daß das fertige Produkt angenehm süß schmeckt.

Die vorgenannten Beispiele sind vorzugsweise abänderbar, indem bis zu 2 TGT, vorzugsweise aber nur bis zu 1 TGT, Saccharose oder ein anderer Zucker, der keine Galactose ist, zugesetzt wird. Höhere Gehälter an Saccharose oder anderem Zucker, der keine Galactose ist, sind möglich aber nicht bevorzugt, weil diese Zucker die Kariesbildung fördern.

Die vorgenannten Beispiele sind weiter abänderbar, indem die eingesetzte Menge an Galactose geändert wird und die übrigen Inhaltsstoffe wie in den vorgenannten Beispielen angegeben belassen werden. Nach diesen Abänderungen kommen beispielsweise vorteilhaft in Betracht:

Bei Beispiel 1: 3, 1 oder 10 TGT Galactose,
bei Beispiel 2: 20, 100 oder 200 TGT Galactose,
bei Beispiel 3: 95, 300 oder 760 TGT Galactose,
bei Beispiel 4: 10, 1 oder 100 TGT Galactose,
bei Beispiel 6: 3, 100 oder 200 TGT Galactose und
bei Beispiel 7: 20, 50 oder 150 TGT Galactose,
jeweils bezogen auf 100 TGT Trägersubstanz, und
bei Beispiel 5: 10, 1 oder 7 TGT Galactose,
bezogen auf 100 ccm Wasser.

Die vorgenannten Beispiele sind weiterhin abänderbar, indem man statt der vorgesehenen reinen Galactose eine entsprechende Menge eines Lactosehydrolysationsproduktes oder eines Molkehydrolysationsproduktes einsetzt. Im Falle eines Molkehydrolysationsproduktes empfiehlt es sich, diesem vor dem Einsatz das Protein zu entziehen, weil das Molkeprotein un ter Umständen die angestrebte Galactosewirkung behindern könnte.

Ein Lactose- oder Molkehydrolysationsprodukt enthält neben der Galactose noch Lactose und Glucose. An sich haben diese Zucker kariesfördernde Wirkung. Man kann die beiden Zucker in den fraglichen Mengen im Schutzmittel aber vertreten, weil sie sich zahnschädigend nicht auswirken können wegen der Galactose, weil sie geschmacksfördernd sind und weil die Galactose in Form eines Lactosehydrolysationsproduktes, insbesondere in Form eines Molkehydrolysationsproduktes, besonders preisgünstig zur Verfügung steht.

## Ansprüche

1. Schutzmittel gegen Karies, dadurch gekennzeichnet,
daß einem zur oralen, humanen Applikation verträglichen Träger, der den Benutzer zu einer längeren Verweilzeit im Mund zwingt, Galactose in feinster Verteilung beigemischt ist.

2. Schutzmittel nach Anspruch 1, dadurch gekennzeichnet,
daß der Gehalt des Trägers an Saccharose 0 bis 2, vorzugsweise 0 TGT auf 100 TGT Trägersubstanz beträgt.

3. Schutzmittel nach Anspruch 1 oder 2, dadurch gekennzeichnet,
daß der Gehalt des Trägers an Zucker, ausgenommen Galactose, 0 bis 2, vorzugsweise 0 TGT auf 100 TGT Trägersubstanz beträgt.

4. Schutzmittel nach einem der vorhergehenden Ansprüche, dadurch gekennzeichnet, daß der Träger eine Zahnpasta, ein Mundwasser, ein Kaugummi oder dergleichen Nicht-Nahrungsmittel ist.

5. Schutzmittel nach Anspruch 4, dadurch gekennzeichnet, daß der Träger eine Zahnpasta ist und daß 1 bis 10 TGT, vorzugsweise 3 TGT, Galactose auf 100 TGT Trägersubstanz eingesetzt sind.

6. Schutzmittel nach Anspruch 5, dadurch gekennzeichnet, daß der Träger aus 40 bis 50 TGT Putzkörper, vorzugsweise feinstkörnigem Kalziumcarbonat, 10 bis 30 TGT Feuchthaltemittel, vorzugsweise Glycerin, 1 bis 2 TGT Binde- und Verdickungsmittel, vorzugsweise Zelluloseester, 0,5 bis 2 TGT Tenside, vorzugsweise Natriumlaurylsulfat, 1 bis 2 TGT sonstige Wirkstoffe, vorzugsweise Vitamine und Mineralsalze, 1 bis 2 TGT Aromastoffe, vorzugsweise ätherische Öle, 0,1 bis 1 TGT Süßstoffe, vorzugsweise Saccharin-Natrium, 0,1 bis 1 TGT Konservierungsstoffe, vorzugsweise Benzoesäure, und gegebenenfalls 1 bis 15 TGT weiteren Zusätzen, wie Binde- und Verdickungsmitteln, Tenside, Wirkstoffe, Aromastoffe, Süßstoffe und Konservierungsstoffe, besteht.

7. Schutzmittel nach Anspruch 4, dadurch gekennzeichnet, daß der Träger aus Kaugummi besteht und daß 50 bis 400, vorzugsweise 300, TGT Galactose auf 100 TGT Trägersubstanz eingesetzt sind.

8. Schutzmittel nach Anspruch 7, dadurch gekennzeichnet, daß der Träger aus Chiclegummi und geschmacksbildenden Zusatzstoffen, wie Eukalyptus-Öl, Süßstoff, Fenchel-Öl, Balsam und dergleichen besteht.

9. Schutzmittel nach Anspruch 4, dadurch gekennzeichnet, daß der Träger Wasser, vorzugsweise Mundwasser, ist und daß 1 bis 10, vorzugsweise 5 TGT Galactose auf 100 ccm (Kubikzentimeter) Wasser gelöst sind.

10. Schutzmittel nach einem der Ansprüche 1 bis 3, dadurch gekennzeichnet, daß der Träger eine Kautablettenmasse ist und daß 3 bis 200 TGT, vorzugsweise 100 TGT, Galactose auf 100 TGT Trägersubstanz eingesetzt sind.

11. Schutzmittel nach Anspruch 6, dadurch gekennzeichnet, daß der Träger im wesentlichen aus Zucker und/oder aus Zuckeraustauschstoff und einem Direktkomprimiermittel, vorzugsweise Calciumhydrogenphosphat, besteht und mit der Galactose vermischt tablettiert ist.

12. Schutzmittel nach einem der Ansprüche 1 bis 3, dadurch gekennzeichnet, daß der Träger ein Lutschbonbon vorwiegend aus Zuckeraustauschstoff, wie Sorbit, Xylit, Manit oder dergleichen, ist und daß 1 bis 100, vorzugsweise 10 TGT Galactose auf 100 TGT Trägersubstanz eingesetzt sind.